# EUROPEAN PATENT APPLICATION

(11) **EP 1 739 431 A1**
(43) Date of publication of application: **03.01.2007**
(21) Application number: 05733999.6
(22) Date of filing: 25.04.2005
(51) Int. Cl.: G01N 33/92, G01N 33/15, G01N 33/50

(54) **METHOD OF JUDGING NON-ALCOHOLIC STEATOHEPATITIS**

(30) Priority: 23.04.2004 JP 2004128458
(71) Applicant: Ajinomoto Co., Inc., Tokyo 104-8315 (JP)
(72) Inventor: TAKATSUKI, Fumihiko, c/o AJINOMOTO CO., INC., Kawasaki-shi, Kanagawa 210-8681 (JP); UENO, Takato, Kurume-shi, Fukuoka 830-0003 (JP); SATA, Michio, Fukuoka-shi, Fukuoka 814-0002 (JP)
(74) Representative: Nicholls, Kathryn Margaret
(86) International application number: PCT/JP2005/007806
(87) International publication number: WO 2005/109006

(57) **Abstract**

According to the present invention, phospholipids of the peripheral blood and fatty acid compositions thereof are measured. Especially, the amounts of phosphatidylserine/phosphatidylinositol and phosphatidyl choline, and fatty acid compositions in the peripheral blood are measured. Thus, the present invention can provide a method for evaluating therapeutic agents of NASH that are useful in the patients with NASH, a method of judging NASH, and the therapeutic agents thereof.

## Description

### Technical Field of the Invention

The present invention relates to methods of judging non-alcoholic steatohepatitis, wherein phospholipids and fatty acid compositions of the phospholipids are used as indexes; methods for searching therapeutic agents of non-alcoholic steatohepatitis, wherein phospholipids and fatty acid compositions thereof are used as indexes; and therapeutic agents of non-alcoholic steatohepatitis.

### Background of the Invention

The changes in lifestyle such as dietary life increase lifestyle-related diseases such as obesity, diabetes and hyperlipidemia. Fatty liver is seen in patients with these lifestyle-related diseases and potential patients, and it is assumed that the frequency of its occurrence is about 20% among them. Until now, it is considered that non-alcoholic fatty liver is the disease having a good prognosis. However, in recent years, it has been clarified that 2 to 3% of such fatty liver develops non-alcoholic steatohepatitis (hereinafter referred to as NASH), wherein, via inflammatory cell infiltration, hepatic parenchyma disorder and fibrosis together with fat deposition in hepatic parenchyma, hepatic cirrhosis develops (Non-patent Literature 1). This disease is the hepatic disease excluding viral and autoimmune hepatic diseases, and characterized by having similar hepatic histological changes as those of alcoholic hepatic diseases. The disease is an important clinical entity because it may progress to hepatic impairment and fibrosis from ordinary fatty liver. The previous reports indicate involvement of leptin, cytokines such as TNF-α, lipids peroxide and free fatty acids. On the other hand, in recent years, it has been reported that the increase of free fatty acids relates to insulin resistance and development of fatty liver, which are behind NASH; and specific lipids have cytotoxicity and act as ligands of immune T-cell receptors and G protein-coupled receptors (GPCR). Further, it is known that changes in fatty acid compositions of lipid cardiolipin that abundantly exist in mitochondria affect the energy exchange efficiency of molecules in the electron transport system. There is a high possibility that the changes in fatty acid compositions of specific fatty acids or complex lipids become a cause of NASH, through the increase of hepatic parenchymal cell disorder, induction of inflammatory reaction and decrease of mitochondrial function. It is considered that NASH develops through two steps (2 hit theory) (Non-patent Literature 2). First, triacylglyceride metabolism is altered by hyperinsulinemia and the like, and fatty liver occurs (1st hit). Then, it is thought that some 2nd hit is given thereto to induce the inflammatory reaction, decrease in the mitochondrial function, cells degeneration, and fibrosis. However, as for the 2nd hit, though there are oxidant stress, a direct toxicity of fatty acids, and endotoxin as candidates thereof, the real one thereof is still unknown.

Further, in hyperinsulinemia and obesity that are background factors of NASH, the analysis report of Younsi M, et al. regarding membrane lipid components is known from the viewpoints of rheologic change and morphology change of red blood cells (Non-patent Literature 3). However, though there are reports on changes of fatty acid compositions in NASH cases, there is no comparative analysis of fatty acid compositions per each complex lipid, which is a functional unit of lipids. Therefore, it has been difficult to obtain the information useful in finding methods for searching therapeutic agents of NASH.

At present, the definite diagnosis between NASH and fatty liver is liver biopsy, but it cannot be conducted routinely because the burden and the risk are too high. An easy and convenient method of judging NASH with peripheral blood and the like has been eagerly desired in order to diagnose NASH promptly and start exercise therapy and nutrition therapy. If lipids or fatty acid compositions thereof that specifically fluctuate in the peripheral blood of patients with NASH can be found, they can be widely applied to as an easy and convenient diagnostic method.
[Non-patent Literature 1] Gastroenterology 121; 710-723
[Non-patent Literature 2] Gastroenterology 114: 842-845
[Non-patent Literature 3] Metabolism 51: 1261-1268
[Non-patent Literature 4] Am J Gastroenterol.; 94(9): 2467-74. (1999)

### Disclosure of the Invention

The object of the present invention is to create methods for evaluating therapeutic agents of NASH; methods of judging NASH with peripheral blood; and therapeutic agents of NASH, wherein lipids that specifically occur in patients with NASH and the changes in the lipid compositions are used.

Since the trigger of NASH is unknown and no animal model exists, the inventors took blood samples from two patients with NASH which were histologically diagnosed (Non-patent Literature 4) and four healthy individuals during the fasting state. Then, they separated plasma and blood cells from the blood samples, and determined change of various lipids and fatty acid compositions thereof. As a result, they found a significant decrease in the amounts of phosphatidylserine/phosphatidylinositol and phosphatidyl choline in the blood cells of the patients with NASH as compared with those of the healthy individuals. Further, the increase in the ratio of fatty acids, 16:00, 18:1n9, and 20:3n3, in the blood cell cardiolipin of the patients with NASH, and the decrease in the ratio of fatty acids, 22:5n3, 22:6n3, and 20:4n6, in their blood cell cardiolipin were observed. The same tendency was observed in the blood cell phosphatidylcholine and the plasma diacylglyceride, and the present invention has been completed based of these findings. Namely, the present invention provides methods of judging NASH with peripheral blood; methods for evaluating therapeutic agents of NASH; and therapeutic agents of NASH.

The present invention provides a method of judging non-alcoholic steatohepatitis, which comprises the step of measuring phospholipids of the peripheral blood and/or the fatty acid compositions thereof. According to this invention, it further provides the method of judging non-alcoholic steatohepatitis, which comprises the steps of measuring the amounts of phosphatidylserine/phosphatidylinositol and phosphatidylcholine in the peripheral blood; and considering it as nonalcoholic steatohepatitis when the value of at least either one of them is lower than a normal value. According to this invention, it also provides the method of judging no-nalcoholic steatohepatitis, wherein the peripheral blood is a blood cell component.

Besides, the present invention provides a method of judging non-alcoholic steatohepatitis, wherein the fatty acids, 16:00, 18:1n9, and 20:3n3, in cardiolipin in the blood cells of the peripheral blood are compared with the normal values and either one of their increases is set as an index; or the fatty acids, 22:5n3, 22:6n3, and 20:4n6, in the cardiolipin are compared with the normal values and either one of their decreases is set as an index.

In addition, the present invention provides a method of judging non-alcoholic steatohepatitis, wherein the fatty acids, 16:00, 18:1n9, and 20:3n3, in phosphatidylcholine in the blood cells of the peripheral blood are compared with the normal values and either one of their increases is set as an index; or the fatty acids, 22:5n3, 22:6n3, and 20:4n6, in the phosphatidylcholine are compared with the normal values and either one of their decreases is set as an index.

Further, the present invention provides a method of judging non-alcoholic steatohepatitis, wherein the fatty acids, 16:00, 18:1n9, and 20:3n3, in diacylglyceride in the plasma are compared with the normal values and either one of their increases is set as an index; or the fatty acids, 20:5n3, 22:6n3, and 20:4n6, in the diacylglyceride are compared with the normal values and either one of their decreases is set as an index. Meanwhile, at least either one or more of the above judging methods may be combined with the other judging method(s).

Besides, the present invention provides a diagnostic equipment for diagnosing non-alcoholic steatohepatitis wherein either thin-layer chromatography, gas chromatography or mass spectrum graphy is combined, and said apparatus which detects from patients' peripheral blood samples the decrease of the amount of phosphatidylserine/phosphatidylinositol or phosphatidylcholine to the normal value thereof; or the increase of fatty acids compositions, 16:00, 18:1n9 and 20:3n3, of polyunsaturated fatty acid compositions in cardiolipin, phosphatidylcholine and diacylglyceride as compared with the normal values thereof, or the decrease of their fatty acid compositions, 22:5n3, 20:5n3, 22:6n3 and 20:4n6, as compared with the normal values thereof.

The present invention also provides a method for searching therapeutic agents (preferably agents for treating non-alcoholic steatohepatitis), which comprises the step of selecting the agent that recovers, at least one or more abnormal value(s) of: the amount of phosphatidylserine/phosphatidylinositol or phosphatidylcholine; the fatty acid compositions, 16:00, 18:1n9, 20:3n3, 22:5n3, 22:6n3, and 20:4n6, in cardiolipin; the fatty acid compositions, 16:00, 18:1n9, 20:3n3, 22:5n3, 22:6n3, and 20:4n6, in phosphatidylcholine; and the fatty acid compositions, 16:00, 18:1n9, 20:3n3, 20:5n3, 22:6n3, and 20:4n6, in diacylglyceride, in the blood cells or plasma of the peripheral blood, to the normal value(s) by administering a test drug in vivo.

Here, it is preferable to set the activity of the enzymes that relate to synthesis and decomposition of phosphatidylserine/phosphatidylinositol and phosphatidylcholine as an index. Further, it is preferable in the mammalian cell culture system to set the changes of the enzymatic activities that relate to synthesis and degradation of phosphatidylserine/ phosphatidylinositol and phosphatidylcholine as an index. It is also preferable in the mammalian cell culture system to set the alteration of gene expression of the enzymes that relate to synthesis and decomposition of phosphatidylserine/phosphatidylinositol and phosphatidylcholine as an index. In addition, it is preferable to set the activity of the enzymes that relate to acylation exchange reaction of cardiolipin, phosphatidylcholine and diacylglyceride as an index.

The present invention additionally provides a therapeutic agent of non-alcoholic steatohepatitis that is diagnosed as positive by using the above searching method, which includes either compound of hormones, biological small proteins, known low-molecular drugs, or low-molecular compounds as an active ingredient.

Further, the present invention provides a therapeutic agent of non-alcoholic steatohepatitis which includes the compound(s) as an active ingredient, such as those having the function for recovering, at least one or more abnormal value(s) of: the amount of phosphatidylserine/phosphatidylinositol or phosphatidy lcholine; the fatty acid compositions, 16:00, 18:1n9, 20:3n3, 22:5n3, 22:6n3, and 20:4n6, in cardiolipin; the fatty acid compositions, 16:00, 18:1n9, 20:3n3, 22:5n3, 22:6n3, and 20:4n6, in phosphatidylcholine; and the fatty acid compositions, 16:00, 18:1n9, 20:3n3, 20:5n3, 22:6n3, and 20:4n6, in diacylglyceride, in the blood cells or plasma of the peripheral blood, to the normal value(s) by administering a test drug in vivo.

### Best Mode for Carrying out the Invention

Phospholipids of the peripheral blood used in the judging methods of the present invention are publicly known ones such as phosphatidylserine, phosphatidylinositol, phosphatidylcholine, lysophosphatidylcholine, phosphatidylethanolamine, sphingomyeline, and cardiolipin. At present, the phospholipids of which measuring method is established are mainly used in the present invention, but they include those of which measuring method will be established in the future. Preferably, they include combination of phosphatidylserine and phosphatidylinositol (phosphatidylserine/phosphatidylinositol, that is, mixed one), phosphatidyl choline and cardiolipin. As mentioned above, as for the measurement of these phospholipids of the peripheral blood, those already established can be used.

Fatty acids in the phospholipids of the peripheral blood used in the judging methods of the present invention include various peripheral saturated or unsaturated fatty acids (considering fatty acid residues as fatty acids) that constitute the phospholipids. At present, those fatty acids of which measuring method is established are mainly used in the present invention, but they include those of which measuring method will be established in the future. The objective fatty acids include fatty acids, 16:00, 18:1n9, 20:3n3, 22:5n3, 22:6n3, and 20:4n6, in cardiolipin; fatty acids, 16:00, 18:1n9, 20:3n3, 22:5n3, 22:6n3, and 20:4n6, in phosphatidylcholine; and fatty acids, 16:00, 18:1n9, 20:3n3, 20:5n3, 22:6n3, and 20:4n6, in diacylglyceride.

The determination in the judging methods of the present invention can be conducted by measuring the phospholipids and/or fatty acids in these phospholipids or fatty acids in the peripheral blood; and comparing the values with those of healthy individuals (normal value), or evaluating changes thereof.

For example, when setting the combination of phosphatidylserine and phosphatidylinositol (phosphatidylserine/phosphatidylinositol) that is a phospholipid in the peripheral blood and phosphatidylcholine as the indexes, a patient can be diagnosed as NASH in case that his or her value is lower than the normal value. In this case, it is preferable to use the blood cell component than whole blood.

Further, when setting cardiolipin that is a phospholipid in the blood cells of the peripheral blood as the index, a patient can be diagnosed as NASH in case that either one of his or her fatty acids, 16:00, 18:1n9, or 20:3n3, in cardiolipin increases as compared with each normal value by measuring them; or either one of his or her fatty acids, 22:5n3, 22:6n3, or 20:4n6, in cardiolipin decreases as compared with each normal value by measuring them.

When setting phosphatidylcholine that is a phospholipid in the blood cells of the peripheral blood as the index, a patient can be diagnosed as NASH in case that either one of his or her fatty acids, 16:00, 18:1n9, or 20:3n3, in phosphatidylcholine increases as compared with each normal value by measuring them; or either one of his or her fatty acids, 22:5n3, 22:6n3, or 20:4n6, in phosphatidylcholine decreases as compared with each normal value by measuring them.

Besides, when setting diacylglyceride that is a phospholipid in the blood cells of the peripheral blood as the index, a patient can be diagnosed as NASH in case that either one of his or her fatty acids, 16:00, 18:1n9, or 20:3n3, in diacylglyceride increases as compared with each normal value by measuring them; or either one of his or her fatty acids, 20:5n3, 22:6n3, or 20:4n6, in diacylglyceride decreases as compared with each normal value by measuring them.

Meanwhile, any judging methods may be optionally combined with each other in the present invention.

A laboratory test apparatus for diagnosing non-alcoholic steatohepatitis is the automated apparatus wherein either of publicly known thin-layer chromatography, gas chromatography or mass spectrum graphy is combined with the above judging method(s), and said apparatus which detects the decrease of the amount of phosphatidylserine/phosphatidylinositol or phosphatidylcholine in whole blood or the blood cells component obtained from patients' peripheral blood samples as compared with the normal value thereof and compares it with data of healthy individuals obtained in advance; or said apparatus which measures variation of the polyunsaturated fatty acid compositions (lipid compositions) in cardiolipin, phosphatidylcholine and diacylglyceride and can conduct the analysis of such data on computer programs. The apparatus can be produced by combining the publicly known arts.

The methods for searching therapeutic agents of NASH of the present invention can be accomplished by screening the compounds that change the phospholipids or the fatty acid compositions thereof in accordance with the above judging methods. For instance, the search can be conducted by observing changes of the above indexes in the mammalian cell culture system and searching compounds that recover at least one or more abnormal value(s) to the normal value(s). As an example, the search can be conducted by setting the activity of the enzymes that relate to acylation exchange reaction of cardiolipin, phosphatidylcholine and diacylglyceride as an index.

As for the other searching methods, the objective compound(s) can be obtained by screening the compounds that have the function for recovering, by administration thereof in vivo, at least one or more abnormal value(s) of: the amount of phosphatidylserine/phosphatidylinositol or phosphatidyl choline; the fatty acid compositions, 16:00, 18:1n9, 20:3n3, 22:5n3, 22:6n3, and 20:4n6, in cardiolipin; the fatty acid compositions, 16:00, 18:1n9, 20:3n3, 22:5n3, 22:6n3, and 20:4n6, in phosphatidylcholine; and the fatty acid compositions, 16:00, 18:1n9, 20:3n3, 20:5n3, 22:6n3, and 20:4n6, in diacylglyceride, in the blood cells or plasma of the peripheral blood, to the normal value(s).

More concretely, it is possible to select the objective compound (drug) by the method comprising the steps of cultured hepatic parenchymal cells of mammals (preferably human or rats) on the 24-well plate in accordance with the typical method; adding various drugs thereto and incubating them 24 hours; and setting the amount of phosphatidylserine/phosphatidylinositol or phosphatidylcholine, and each fatty acids, 22:5n3, 22:6n3, and 20:4n6, of phosphatidylserine/phosphatidylinositol, phosphatidylcholine and cardiolipin in the cell membrane after completion of the incubation, as the indexes.

In the above method, it is also possible in the similar culture system to conduct screening of drugs by extracting RNA from the hepatic parenchymal cells after completion of the cultivation, and setting the expression levels of delta-5 desaturase, delta-6 desaturase, elongase, oxidase, and acyltransferase as the indexes. More concretely, variation of gene expression such as that of delta-5 desaturase, delta-6 desaturase, elongase, oxidase, lecithin:cholesterol acyltransferase, lecithin retinol acyltransferase, sn-glycerol 3-phosphate acyltransferase, 1-acyl-sn-glycerol-3-phosphate acyltransferase, phosphocholine cytidylyltransferase, phoshplipase A1 and A2 can be set as the indexes by using the typical method of RT-PCR.

Therapeutic agents of NASH obtained by these searching methods include hormones, biological small proteins, known low-molecular drugs, and low-molecular compounds.

These therapeutic agents of NASH can be administered by optionally combining the dosage, administration methods, and dosage forms corresponding to the symptoms. Here, the dosage forms can be optionally determined by combining publicly known arts.

It is also possible to use these therapeutic agents in combination with other drugs having therapeutic effects on NASH such as an ursodeoxycholic acid, betaine, metformin, glitazone, fibrates, vitamins, antioxidant agents, anti-inflammatory agents, antidiabetic agents, antihyperlipidemic agents and anti-obesity agents. The administered dose thereof is not particularly limited, and it is preferable to administer to patients in the form of drugs containing lug to 100g of the active ingredient.

The present invention provides methods of judging patients with NASH, apparatuses of judging them, methods for evaluating therapeutic agents of NASH and therapeutic agents of NASH.

### Examples

### Example 1 Method for analyzing lipids/fatty acids of NASH

Four healthy individuals and two patients that were definitely diagnosed as NASH by liver biopsy based on the report of Brunt E. M., et al. (Non-patent Literature 4) were included in the study. 5ml of venous blood was taken through the arm vein using a test tube with EDTA during the fasting state. After stirring the test tube well, the number of the red blood cells (RBC) was counted. Then, the blood samples were centrifuged (3000rpm, room temperature, 5 mins.) and the plasma was collected and stored at -80°C until the analysis. Pellets of RBC in the tube were washed well with saline solution with EDTA, 1x10⁹ of RBC were obtained and centrifuged (3000rpm, room temperature, 5 mins.). The RBC was stored at -80°C until the analysis. The lipids of the plasma and RBC were extracted by the Folch's method. First, each lipid was separated by a thin-layer chromatography. Each SPOT of the lipids was collected and treated with 3N methanol / hydrochloric acid at 100°C for 45 minutes under filling nitrogen gas. The isolated methylester of fatty acids was extracted using hexane containing 0.05% butylated hydroxytoluene under nitrogen gas. Then, the methylester of fatty acids was separated with a gas chromatography and the quantity thereof was determined with a flame-ionization detector based on each preparation sample of methylester of fatty acids.

Results are shown in Tables 1 and 2. As the tables clarify, the decrease in the amounts of phosphatidylserine/phosphatidylinositol and phosphatidylcholine in the blood cells was observed in the patients with NASH. The increase in fatty acids, 16:00, 18:1n9, and 20:3n3, and the decrease in fatty acids, 22:5n3, 22:6n3, and 20:4n6, in the blood cell cardiolipin were also observed in the patients with NASH. The similar tendency was observed in the red blood cell phosphatidylcholine and the plasma diacylglyceride, and, therefore, it became possible to differentiate the patients with NASH based on these indexes.

**Table 1**

| Variation of lipid composition | Normal 1 | Normal 2 | Normal 3 | Normal 4 | NASH 1 | NASH 2 |
|---|---|---|---|---|---|---|
| RBC (nM/g) | | | | | | |
| Phosphatidylserine/Inositol | 1131.2 | 1181.4 | 1342.6 | 988.2 | 564.0 | 515.7 |
| Phosphatidylethanolamine | 565.8 | 418.2 | 366.6 | 357.2 | 396.5 | 284.3 |
| Phosphatidylcholine | 1251.3 | 1648.0 | 1446.9 | 2300.5 | 938.2 | 565.6 |
| Lysophosphatidylcholine | 113.4 | 105.2 | 93.4 | 115.6 | 103.8 | 73.8 |
| Cardiolipin | 467.9 | 256.6 | 235.5 | 273.0 | 240.4 | 241.3 |

| Blood plasma (nM/g) | | | | | | |
|---|---|---|---|---|---|---|
| Phosphatidylserine/Inositol | | | | | | |
| Phosphatidylethanolamine | 378.3 | 237.6 | 370.1 | 341.6 | 333.7 | 199.3 |
| Phosphatidylcholine | 1319.5 | 1313.0 | 1668.7 | 1164.3 | 1059.8 | 1318.8 |
| Lysophosphatidylcholine | 210.2 | 186.3 | 208.6 | 196.0 | 166.7 | 182.6 |
| Cardiolipin | | | | | | |
| Triacylglcyeride | 388.2 | 318.6 | 668.5 | 573.8 | 970.9 | 1319.2 |

**Table 2**

| Variation of FA composition | Normal | 1 Normal | 2 Normal | 3 Normal 4 | NASH 1 | NASH 2 |
|---|---|---|---|---|---|---|
| RBC (%) | | | | | | |
| Cardiolipin 16:00 | 15.4 | 11.2 | 10.9 | 127 | 17.1 | 17.5 |
| Cardiolipin 18:1n9 | 14.1 | 11.7 | 11.8 | 11.7 | 15.0 | 14.5 |
| Cardiolipin 20:3n3 | 0.2 | 0.2 | 0.5 | 0.7 | 1.3 | 1.0 |
| Cardiolipin 22:5n3 | 2.7 | 3.0 | 4.5 | 3.8 | 2.6 | 2.8 |
| Cardiolipin 22:6n3 | 9.6 | 11.3 | 14.6 | 14.7 | 11.1 | 10.3 |
| Cardiolipin 20:4n8 | 15.5 | 19.0 | 19.5 | 18.0 | 15.8 | 17.0 |
| Phosphatidylcholine 16:00 | 37.2 | 34.3 | 35.8 | 30.5 | 37.6 | 36.2 |
| 18:1n9 | 12.0 | 10.5 | 14.0 | 13.0 | 15.8 | 15.2 |
| 20:3n3 | 0.0 | 0.0 | 0.0 | 0.0 | 0.0 | 0.0 |
| 22:5n3 | 0.7 | 1.0 | 0.4 | 0.6 | 0.0 | 0.0. |
| 22:6n3 | 3.8 | 5.5 | 3.1 | 3.7 | 2.9 | 3.4 |
| 20:4n6 | 9.2 | 9.0 | 7.8 | 7.4 | 7.2 | 7.6 |

| Blood plasma (%) | | | | | | |
|---|---|---|---|---|---|---|
| Diacylglcyeride 16:00 | 20.1 | 17.3 | 28.4 | 26.4 | 30.6 | 26.3 |
| Diacylglcyeride 18:1n9 | 5.9 | 9.8 | 14.9 | 15.7 | 17.1 | 12.1 |
| Diacylglcyeride 20:3n3 | 1.5 | 0.6 | 1.0 | 0.0 | 0.8 | 0.9 |
| Diacylglcyeride 20:5n3 | 1.8 | 1.4 | 0.0 | 0.0 | 0.0 | 0.0 |
| Diacylglcyeride 22;8n3 | 2.8 | 1.3 | 3.0 | 4.3 | 0.0 | 0.0 |
| Diacylglcyeride 20:4n6 | 0.8 | 0.5 | 0.0 | 1.7 | 0.0 | 0.0 |

According to the judging methods and the apparatuses of the present invention, it has become possible to identify the patients with NASH. Similarly, according to the methods for searching therapeutic agents of NASH of the present invention, it has become possible to search therapeutic agents of NASH, and, therefore, the present invention is useful.

## Claims

1. A method of judging non-alcoholic steatohepatitis, which comprises the step of measuring phospholipids or fatty acid compositions of the phospholipids in the peripheral blood.

2. The method of judging non-alcoholic steatohepatitis according to claim 1, which comprises the steps of measuring the amounts of phosphatidylserine/phosphatidylinositol and phosphatidylcholine in the peripheral blood; and considering it as non-alcoholic steatohepatitis when the value of at least either one of them is lower than a normal value.

3. The method of judging non-alcoholic steatohepatitis according to claim 2, wherein the peripheral blood is a blood cell component.

4. A method of judging non-alcoholic steatohepatitis, wherein the fatty acids, 16:00, 18:1n9, and 20:3n3, in cardiolipin in the blood cells of the peripheral blood are compared with the normal values and either one of their increases is set as an index.

5. A method of judging non-alcoholic steatohepatitis, wherein the fatty acids, 22:5n3, 22:6n3, and 20:4n6, in the cardiolipin in the blood cells of the peripheral blood are compared with the normal values and either one of their decreases is set as an index.

6. A method of judging non-alcoholic steatohepatitis, wherein the fatty acids, 16:00, 18:1n9, and 20:3n3, in phosphatidylcholine in the blood cells of the peripheral blood are compared with the normal values and either one of their increases is set as an index.

7. A method of judging non-alcoholic steatohepatitis, wherein the fatty acids, 22:5n3, 22:6n3, and 20:4n6, in the phosphatidylcholine in the blood cells of the peripheral blood are compared with the normal values and either one of their decreases is set as an index.

8. A method of judging non-alcoholic steatohepatitis, wherein the fatty acids, 16:00, 18:1n9, and 20:3n3, in diacylglyceride in the plasma are compared with the normal values and either one of their increases is set as an index.

9. A method of judging non-alcoholic steatohepatitis, wherein the fatty acids, 20:5n3, 22:6n3, and 20:4n6, in the diacylglyceride in the plasma are compared with the normal values and either one of their decreases is set as an index.

10. A method of judging non-alcoholic steatohepatitis, which comprises the step of combining at least either one or more of the judging method(s) according to any one of claims 4 to 9 with the other judging method(s).

11. A laboratory test apparatus for diagnosing non-alcoholic steatohepatitis, wherein either thin-layer chromatography, gas chromatography or mass spectrum graphy is combined, and said apparatus which detects from patients' peripheral blood samples the decrease of the amount of phosphatidylserine/phosphatidylinositol or phosphatidylcholine to the normal value thereof; or the increase of fatty acids compositions, 16:00, 18:1n9 and 20:3n3, of polyunsaturated fatty acid compositions in cardiolipin, phosphatidylcholine and diacylglyceride as compared with the normal values thereof, or the decrease of their fatty acid compositions, 22:5n3, 20:5n3, 22:6n3 and 20:4n6, as compared with the normal values thereof.

12. A method for searching therapeutic agents, which comprises the step of selecting the agent that recovers, at least one or more abnormal value(s) of the amount of phosphatidylserine/phosphatidylinositol or phosphatidylcholine; the fatty acid compositions, 16:00, 18:1n9, 20:3n3, 22:5n3, 22:6n3, and 20:4n6, in cardiolipin; the fatty acid compositions, 16:00, 18:1n9, 20:3n3, 22:5n3, 22:6n3, and 20:4n6, in phosphatidylcholine; and the fatty acid compositions, 16:00, 18:1n9, 20:3n3, 20:5n3, 22:6n3, and 20:4n6, in diacylglyceride, in the blood cells or plasma of the peripheral blood, to the normal value(s) by administering a test drug in vivo.

13. A therapeutic agent of non-alcoholic steatohepatitis that is diagnosed as positive by using the searching method according to claim 12, which includes either compound of hormones, biological small proteins, known low-molecular drugs, or low-molecular compounds as an active ingredient.
